# EUROPEAN PATENT APPLICATION

(11) **EP 1 790 657 A1**
(43) Date of publication of application: **30.05.2007**
(21) Application number: 05025670.0
(22) Date of filing: 24.11.2005
(51) Int. Cl.: C07K 14/00, A61K 9/127, A61K 47/48, A61K 47/42, C07K 7/08

(54) **pH-switchable transmembrane peptides as stimulators of membrane fusion**

(71) Applicant: Technische Universität München, 80333 München (DE)
(72) Inventor: Langosch, Dieter, Prof. Dr., 85354 Freising (DE); Hofmann, Mathias, 85356 Freising (DE); Gütlich, Markus, Dr., 85405 Nandlstadt (DE)
(74) Representative: Graf von Stosch, Andreas

(57) **Abstract**

This invention relates to pH-switchable transmembrane peptides as well as complexes containing them and their use as stimulators of membrane fusion. The peptides of the invention are particularly useful for transfecting therapeutically active substances into eukaryotic cells.

SEQUENCE LISTING

<110>  Technische Universitaet Muenchen

<120>  pH-switschable transmembrane peptides as stimulators of membrane
       fusion

<130>  TU01P001EP

<140>  EP05025670.0
<141>  2005-11-24

<160>  32

<170>  PatentIn version 3.3

<210>  1
<211>  5
<212>  PRT
<213>  Artificial

<220>
<223>  Description of sequence:  sequence according to formula (I) (see
       description p. 5)


<220>
<221>  misc_feature
<222>  (1)..(1)
<223>  Xaa is an essentially hydrophilic amino acid, preferably glycine
       (G), lysine (K) or arginine (R), more preferably histidine (H)

<220>
<221>  REPEAT
<222>  (1)..(1)
<223>  repeat number is 0 or 1

<220>
<221>  misc_feature
<222>  (2)..(2)
<223>  Xaa is histidine (H);

<220>
<221>  REPEAT
<222>  (2)..(2)
<223>  repeat number is an integer from 1 to 6; or an integer from 0
       to 5; provided that amino acids at positions 1 and 5 are histidine
       (H) and the repeat number thereof is 1

<220>
<221>  misc_feature
<222>  (3)..(3)
<223>  Xaa is an essentially hydrophobic amino acid, preferably selected
       from the group consisting of valine (V), leucine (L), isoleucine
       (I), proline (P), glycine (G), cysteine (C), tryptophane (W),
       alanine (A), phenylalanine (F) and methionine (M); and ,

<220>
<221>  REPEAT
<222>  (3)..(3)
<223>  repeat number is an integer from 7 to 30;

<220>
<221>  misc_feature
<222>  (4)..(4)
<223>  Xaa is histidine (H);

<220>

<221>  REPEAT
<222>  (4)..(4)
<223>  repeat number is an integer from 1 to 6; or an integer from 0
       to 5; provided that amino acids at positions 1 and 5 are histidine
       (H) and the repeat number thereof is 1

<220>
<221>  misc_feature
<222>  (5)..(5)
<223>  Xaa is an essentially hydrophilic amino acid, preferably glycine
       (G), lysine (K) or arginine (R), more preferably histidine (H)

<220>
<221>  REPEAT
<222>  (5)..(5)
<223>  repeat number is 0 or 1

<400>  1

Xaa Xaa Xaa Xaa Xaa
1               5


<210>  2
<211>  4
<212>  PRT
<213>  Artificial

<220>
<223>  Description of sequence: sequence according to formula II (see
       description p. 10)

<220>
<221>  REPEAT
<222>  (1)..(4)
<223>  repeat number for entire sequence is an integer from 1 to 15,
       provided that the core comprises from at least 6 to 30 amino
       acids

<220>
<221>  misc_feature
<222>  (1)..(1)
<223>  Xaa is Lysine (K); or according to an alternative pattern, with
       2, 3 or 4 essentially different hydrophobic amino acids in
       the sequence, wherein Xaa may represent any essentially
       hydrophobic amino acid

<220>
<221>  REPEAT
<222>  (1)..(1)
<223>  repeat number is an integer from 1 to 5

<220>
<221>  misc_feature
<222>  (2)..(2)
<223>  Xaa is Leucine (L); or according to an alternative pattern, with
       2, 3 or 4 essentially different hydrophobic amino acids in
       the sequence, wherein Xaa may represent any essentially
       hydrophobic amino acid

<220>
<221>  REPEAT
<222>  (2)..(2)
<223>  repeat number is an integer from 1 to 5

<220>
<221>  misc_feature
<222>  (3)..(3)

<223>  Xaa is Methionine (M); or according to an alternative pattern, with
       2, 3 or 4 essentially different hydrophobic amino acids in
       the sequence, wherein Xaa may represent any essentially
       hydrophobic amino acid

<220>
<221>  REPEAT
<222>  (3)..(3)
<223>  repeat number is an integer from 0 to 5

<220>
<221>  misc_feature
<222>  (4)..(4)
<223>  Xaa is Asparagine (N); or according to an alternative pattern, with
       2, 3 or 4 essentially different hydrophobic amino acids in
       the sequence, wherein Xaa may represent any essentially
       hydrophobic amino acid

<220>
<221>  REPEAT
<222>  (4)..(4)
<223>  repeat number is an integer from 0 to 5

<400>  2

Lys Leu Met Asn
1


<210>  3
<211>  23
<212>  PRT
<213>  Artificial

<220>
<223>  peptide sequence 3-His-L16 (see Fig. 1)

<400>  3

His His His Trp Leu Leu Leu Leu Leu Leu Leu Leu Leu Leu Leu Leu
1               5                   10                  15


Leu Leu Leu Leu His His His
            20


<210>  4
<211>  23
<212>  PRT
<213>  Artificial

<220>
<223>  peptide sequence 3-His-LV16 (see Fig. 2)

<400>  4

His His His Trp Leu Val Leu Val Leu Val Leu Val Leu Val Leu Val
1               5                   10                  15


Leu Val Leu Val His His His
            20


<210>  5
<211>  23
<212>  PRT

<213>  Artificial

<220>
<223>  peptide sequence 3-His-LV16-G8P9 (see Fig. 3)

<400>  5

His His His Trp Leu Val Leu Val Leu Val Leu Gly Pro Val Leu Val
1               5                   10                  15

Leu Val Leu Val His His His
            20

<210>  6
<211>  23
<212>  PRT
<213>  Artificial

<220>
<223>  peptide sequence 3-His-LLV16 (see Fig. 4)

<400>  6

His His His Trp Leu Leu Val Leu Leu Val Leu Leu Val Leu Leu Val
1               5                   10                  15

Leu Leu Val Leu His His His
            20

<210>  7
<211>  23
<212>  PRT
<213>  Artificial

<220>
<223>  peptide sequence 3-His-VVL16 (see Fig. 5)

<400>  7

His His His Trp Val Val Leu Val Val Leu Val Val Leu Val Val Leu
1               5                   10                  15

Val Val Leu Val His His His
            20

<210>  8
<211>  23
<212>  PRT
<213>  Artificial

<220>
<223>  peptide sequence 3-His-LV16-G8 (see Fig. 6)

<400>  8

His His His Trp Leu Val Leu Val Leu Val Leu Gly Leu Val Leu Val
1               5                   10                  15

Leu Val Leu Val His His His
            20

<210>  9
<211>  23
<212>  PRT
<213>  Artificial

<220>
<223>  peptide sequence 3-His-LV16-P8 (see Fig. 7)

<400>  9

His His His Trp Leu Val Leu Val Leu Val Leu Pro Leu Val Leu Val
1               5                   10                  15

Leu Val Leu Val His His His
            20

<210>  10
<211>  23
<212>  PRT
<213>  Artificial

<220>
<223>  peptide sequence 3-His-LV16-P8G9 (see Fig. 8)

<400>  10

His His His Trp Leu Val Leu Val Leu Val Leu Pro Gly Val Leu Val
1               5                   10                  15

Leu Val Leu Val His His His
            20

<210>  11
<211>  23
<212>  PRT
<213>  Artificial

<220>
<223>  peptide sequence 3-His-LV16-G6P10 (see Fig. 9)

<400>  11

His His His Trp Leu Val Leu Val Leu Gly Leu Val Leu Pro Leu Val
1               5                   10                  15

Leu Val Leu Val His His His
            20

<210>  12
<211>  23
<212>  PRT
<213>  Artificial

<220>
<223>  peptide sequence 3-His-LV16-GPPG (see Fig. 10)

<400>  12

His His His Trp Leu Val Leu Val Gly Pro Leu Val Leu Val Pro Gly
1               5                   10                  15

Leu Val Leu Val His His His
            20

<210>  13
<211>  21
<212>  PRT
<213>  Artificial

<220>
<223>  peptide sequence 2-His-L16 (see Fig. 11)

<400>  13

His His Trp Leu Leu Leu Leu Leu Leu Leu Leu Leu Leu Leu Leu Leu
1               5                   10                  15

Leu Leu Leu His His
            20

<210>  14
<211>  21
<212>  PRT
<213>  Artificial

<220>
<223>  peptide sequence 2-His-LV16 (see Fig. 12)

<400>  14

His His Trp Leu Val Leu Val Leu Val Leu Val Leu Val Leu Val Leu
1               5                   10                  15

Val Leu Val His His
            20

<210>  15
<211>  21
<212>  PRT
<213>  Artificial

<220>
<223>  peptide sequence 2-His-LV16-G8P9 (see Fig. 13)

<400>  15

His His Trp Leu Val Leu Val Leu Val Leu Gly Pro Val Leu Val Leu
1               5                   10                  15

Val Leu Val His His
            20

<210>  16
<211>  21
<212>  PRT
<213>  Artificial

<220>
<223>  peptide sequence 2-His-LLV16 (see Fig. 14)

<400>  16

His His Trp Leu Leu Val Leu Leu Val Leu Leu Val Leu Leu Val Leu
1               5                   10                  15

Leu Val Leu His His
            20

<210>  17
<211>  21
<212>  PRT
<213>  Artificial

<220>
<223>  peptide sequence 2-His-VVL16 (see Fig. 15)

<400>  17

His His Trp Val Val Leu Val Val Leu Val Val Leu Val Val Leu Val
1               5                   10                  15

Val Leu Val His His
            20

<210>  18
<211>  21
<212>  PRT
<213>  Artificial

<220>
<223>  peptide sequence 2-His-LV16-G8 (see Fig. 16)

<400>  18

His His Trp Leu Val Leu Val Leu Val Leu Gly Leu Val Leu Val Leu
1               5                   10                  15

Val Leu Val His His
            20

<210>  19
<211>  21
<212>  PRT
<213>  Artificial

<220>
<223>  peptide sequence 2-His-LV16-P8 (see Fig. 17)

<400>  19

His His Trp Leu Val Leu Val Leu Val Leu Pro Leu Val Leu Val Leu
1               5                   10                  15

Val Leu Val His His
            20

<210>  20
<211>  21
<212>  PRT
<213>  Artificial

<220>
<223>  peptide sequence 2-His-LV16-P8G9 (see Fig. 18)

<400>  20

His His Trp Leu Val Leu Val Leu Val Leu Pro Gly Val Leu Val Leu
1               5                   10                  15

Val Leu Val His His
            20

<210>  21
<211>  21
<212>  PRT
<213>  Artificial

<220>
<223>  peptide sequence 2-His-LV16-G6P10 (see Fig. 19)

<400>  21

His His Trp Leu Val Leu Val Leu Gly Leu Val Leu Pro Leu Val Leu
1               5                   10                  15

Val Leu Val His His
            20

<210>  22
<211>  21
<212>  PRT
<213>  Artificial

<220>
<223>  peptide sequence 2-His-LV16-GPPG (see Fig. 20)

<400>  22

His His Trp Leu Val Leu Val Gly Pro Leu Val Leu Val Pro Gly Leu
1               5                   10                  15

Val Leu Val His His
            20

<210>  23
<211>  19
<212>  PRT
<213>  Artificial

<220>
<223>  peptide sequence 1-His-L16 (see Fig. 21)

<400>  23

His Trp Leu Leu Leu Leu Leu Leu Leu Leu Leu Leu Leu Leu Leu Leu
1               5                   10                  15

Leu Leu His

<210>  24

<211>  19
<212>  PRT
<213>  Artificial

<220>
<223>  peptide sequence 1-His-LV16 (see Fig. 22)

<400>  24

His Trp Leu Val Leu Val Leu Val Leu Val Leu Val Leu Val Leu Val
1               5                   10                  15

Leu Val His

<210>  25
<211>  19
<212>  PRT
<213>  Artificial

<220>
<223>  peptide sequence 1-His-LV16-G8P9 (see Fig. 23)

<400>  25

His Trp Leu Val Leu Val Leu Val Leu Gly Pro Val Leu Val Leu Val
1               5                   10                  15

Leu Val His

<210>  26
<211>  19
<212>  PRT
<213>  Artificial

<220>
<223>  peptide sequence 1-His-LLV16 (see Fig. 24)

<400>  26

His Trp Leu Leu Val Leu Leu Val Leu Leu Val Leu Leu Val Leu Leu
1               5                   10                  15

Val Leu His

<210>  27
<211>  19
<212>  PRT
<213>  Artificial

<220>
<223>  peptide sequence 1-His-VVL16 (see Fig. 25)

<400>  27

His Trp Val Val Leu Val Val Leu Val Val Leu Val Val Leu Val Val
1               5                   10                  15

Leu Val His

<210>  28
<211>  19
<212>  PRT
<213>  Artificial

<220>
<223>  peptide sequence 1-His-LV16-G8 (see Fig. 26)

<400>  28

His Trp Leu Val Leu Val Leu Val Leu Gly Leu Val Leu Val Leu Val
1               5                   10                  15

Leu Val His

<210>  29
<211>  19
<212>  PRT
<213>  Artificial

<220>
<223>  peptide sequence 1-His-LV16-P8 (see Fig. 27)

<400>  29

His Trp Leu Val Leu Val Leu Val Leu Pro Leu Val Leu Val Leu Val
1               5                   10                  15

Leu Val His

<210>  30
<211>  19
<212>  PRT
<213>  Artificial

<220>
<223>  peptide sequence 1-His-LV16-P8G9 (see Fig. 28)

<400>  30

His Trp Leu Val Leu Val Leu Val Leu Pro Gly Val Leu Val Leu Val
1               5                   10                  15

Leu Val His

<210>  31
<211>  19
<212>  PRT
<213>  Artificial

<220>
<223>  peptide sequence 1-His-LV16-G6P10 (see Fig. 29)

<400>  31

His Trp Leu Val Leu Val Leu Gly Leu Val Leu Pro Leu Val Leu Val

1               5                   10                  15

Leu Val His

<210>  32
<211>  19
<212>  PRT
<213>  Artificial

<220>
<223>  peptide sequence 1-His-LV16-GPPG (see Fig. 30)

<400>  32

His Trp Leu Val Leu Val Gly Pro Leu Val Leu Val Pro Gly Leu Val
1               5                   10                  15

Leu Val His

## Description

This invention relates to pH-switchable transmembrane peptides as well as complexes containing them and their use as stimulators of membrane fusion. The peptides of the invention are particularly useful for transfecting eukaryotic cells by therapeutically active substances.

The controlled delivery of therapeutically active substances, in particular drugs, nucleic acids and other pharmaceutical substances, into cells has been a challenge for scientists and researchers in the last twenty years. Therefore, major efforts have been undertaken and many methods have been developed to achieve an effective transport system for the delivery of such substances into cells.

Different modes to administer therapeutically active substances are well known in the art. All of them allow to provide therapeutically active substances in body fluids. If oral administration is to be avoided, parenteral administration of therapeutically active substances including, for example, intravascular, intratumor, intraparenchymal, intradermal, subdermal, subcutaneous, intraperitoneal, intramuscular and intralymphatic injections via a syringe, is preferred. After administration, the therapeutically active substances are dispersed by the blood circulatory system. Thus, effective transport into the cell primarily requires extracellular transport in the blood stream. Accordingly, hydrophilic polymers, such as polyethylene glycol (PEG), may be used to provide protection of the therapeutically active substance during its presence in the blood system. Such polymers may inhibit interaction of therapeutically active substances with blood components or their degradation and may increase the circulatory time of therapeutically active substances by suppressing opsonization and phagocytosis. However, effective transport into target cells is of major importance as well. Effective transport into cells requires after parental administration preferably controlled release of therapeutically active substances (delivered into the e.g. bloodstream) by targeting specific tissues and transport across membranes, e.g. the cell membrane. To enable controlled release, complexes of therapeutically active substances and biologically labile polymers, such as copolymers of poly(lactic/glycolic acid) (PLGA) and ethylvinyl acetate/polyvinyl alcohol, have been designed to delay release of therapeutically active substances (Jain, R. et al. Drug Dev. Ind. Pharm. 24: 703-727 (1998); Metrikin, D.C. et al., Curr. Opin. Ophthalmol. 5: 21-29, 1994).

Another way of administration, which also requires an effective transport into the cell, is based on oral administration. However, in case of oral administration, acidity of the stomach (pH less than 2) hampers already at early stage effective delivery of pH-sensitive or - labile therapeutically active substances. Therefore, capsules have been developed that are based on pH-dependent degradability/solubility of the capsule polymer. The capsules release their contents into the small intestine. Polymers, e.g., copolymers of polymethacrylic acid (Eudragit S, Rohm America), which are insoluble at lower pH but readily soluble at higher pH, can be used as enteric coatings (Zhu et al. J. Drug Target., 7, 223, 1999).

Nevertheless, it is of importance for either way of administration, to ensure an effective transport into the target cell. A successful approach is based on cell transfection. Many biological substances are taken up by cells via receptor-mediated endocytosis (Pastan I. et al., Science 214: 504-509, 1981). This mechanism involves binding of a ligand to a cell-surface receptor, clustering of ligand-bound receptors and formation of coated pits followed by internalization of the ligands into the endosomes. For example, enveloped viruses, e.g., influenza virus and alphaviruses, and non-enveloped viruses, e.g., adenovirus, infect cells via endocytotic pathways (Pastan, I. et al., Am.Soc.Microbiology, Washington, 141-146, 1986; Kielian, M. et al., Plenum Press, New York, 91-119, 1986; FitzGerald, D.J.P. et al., Cell 32: 607-617, 1983).

In general, viruses can be modified to carry a desired gene, and, thus, become a suitable vector for gene therapy. By use of standard recombinant techniques, viral genes can be removed and replaced with the desired gene which may replace its pathogenic analogue in the genome. In particular, various retroviruses were modified in this way, which support current gene therapy efforts. However, retroviruses are not useful to transfect the whole spectrum of different cell types, which have to be transfected efficiently for various disorders. Other viral vectors based on herpes virus were designed to enable more efficient gene transfer into cells, e.g., brain cells. Adenoviral and adenoassociated vectors were developed to infect lung and other cells. However, use of viral vectors for gene therapy has significant drawbacks. This is due to the risk of remaining infectious material of viral origin in modified viruses. Thereby, a therapeutic virus may become infectious for the host to be treated.

Apart from use of viral vectors, polynucleotides, genes etc. have been directly transferred into mammalian cells. In this regard, the desired gene is incorporated into bacterial plasmid DNA together with a mammalian promoter, an enhancer and other sequences enabling, gene expression in mammalian cells after transfection. Plasmid DNA may be complexed with proteins that target plasmid DNA to specific tissues. Plasmid DNA containing the desired gene may also be incorporated into lipid vesicles, e.g. liposomes having suitable lipidic composition, e.g. containing cationic lipids in the lipidic composition such as Lipofectin, which may facilitate transfer of plasmid DNA into target cells.

Liposomes may also support introduction of other macromolecules such as RNA, peptides, proteins or therapeutic agents of low molecular weight into the cell, and are generally used as drug delivery vehicles. Many therapeutically active substances, such as polynucleotides or polypeptides and in particular highly charged compounds, are incapable of crossing cell membranes by themselves. Liposomes function as transporter vehicles and enable the transport of these charged macromolecules into cells via membrane fusion. US 6,376,248 describes liposome-containing compositions useful for transfecting eukaryotic cells. These compositions comprise complexes of (therapeutic) nucleic acids, peptides and transfection agents. The peptides are typically covalently linked to the transfection agent, which may include any lipid aggregate, such as liposomes. The peptides exert either fusogenic or membrane-permeabilizing activity or may be involved in homing to the therapeutically active agents sub-cellular localization, cellular transport or receptor interaction of the liposome complex. Peptides of US 6,376,248 may also be used for binding to lipidic components. However, effects on their transporter properties due to a pH gradient between various cell compartments is not taken into account by the complexes disclosed in US 6,376,248. They are generally incorporated by cells via endocytosis channeling the peptides into endosomes. However, the endosomal pH of 5 to 6 is significantly lower than the cytosolic pH. At pH 5 to 6 an efficient fusion between the anionic lipid doped liposomal membranes (transfection agent) and the anionic surface of the endosomal membrane is unlikely to occur because of charge repulsion. As a result, liposomal carriers according to US 6,376,248, which are transported along the endosomal pathway are prone to degradation in lysosomes. Thereby, the lipid complex and its therapeutically active component is subjected to enzymatic degradation. Accordingly, the therapeutic effect is significally diminished.

US 6,610,664 discloses complexes of cationic lipids and nucleic acids, oligonucleotides or other therapeutic agents as delivery systems. Cationic lipids have up to 3 charged moieties in their charged domain. Thereby, interaction between the cationic lipid and the negatively charged inner surface of an endosome is intended to be increased. However, cationic lipids, as disclosed in US 6,610,664, are not capable to protect therapeutically active agents from enzymatic degradation in various cell compartments - in contrast to liposomes, which enclose a therapeutically active agent. Moreover, use of cationic lipids as vehicle of biologically or therapeutically active substances has been associated with induction of apoptosis (Picotti JR et al., Drug Chem. Toxicol., vol. 28(1), 117-133, 2005), which affects their applicability in pharmacy.

In order to overcome the problems involved with transport of therapeutics along a pH gradient, US 6,630,351 discloses cell delivery of therapeutically active compounds, such as nucleic acids and drugs, using a pH-sensitive delivery system. Polymers and therapeutically active molecules are coupled to membrane active compounds. Polymers as well as membrane active compounds contain pH labile bonds, which may be cleaved under cellular acidic pH conditions. Cleavage of these pH labile bonds may result in an increase of membrane activity combined with locally altered membrane structures. However, activity of membrane active compounds as disclosed by US 6,630,351 has to be controlled by another component, namely an inhibitory compound. Therefore, this system is complex due to a control mechanism required for the regulation of membrane active compounds. Moreover, the drug delivery system according to US 6,630,351 is not based on the principle of membrane fusion. Finally, the approach according to US 6,630,351 requires protein complexes of high molecular weight, which are expensive in production and susceptible to proteolytic degradation and denaturation.

In summary, cell transfection systems described in the art either neglect structural or chemical transformation of therapeutic compounds to be delivered into the cell due to differing pH conditions in various extracellular or intracellular compartments. Differing pH conditions usually prevent effective delivery of pH-sensitive or labile therapeutically active substances into cells. Or, if pH dependency is taken into account for state-of-the-art delivery systems, they do not allow their therapeutically active cargo to be protected from degradation along the entry route to their target site.

It is therefore an object of the invention to provide a cost-effective and efficient cell transfection system therapeutically active substances, such as pharmaceuticals and nucleic acids, whereby varying pH conditions along the cell entry route are taken into account.

The invention relates in its first embodiment to a peptide having an amino acid sequence of formula I:

(A)ₓ(B)_{y}(C)_{z}(B)_{y}(A)ₓ

wherein
- A: is an essentially hydrophilic amino acid, preferably glycine (G), lysine (K) or arginine (R), more preferably histidine (H);
- X: is 0 or 1;
- B: is histidine (H);
- Y: is an integer from 1 to 6; or
- Y: is an integer from 0 to 5; provided that A is histidine (H) and X is 1;
- C: is an essentially hydrophobic amino acid, preferably selected from the group consisting of valine (V), leucine (L), Isoleucine (I), proline (P), glycine (G), cysteine (C), tryptophane (W), alanine (A), phenylalanine (F) and methionine (M); and
- Z: is an integer from 7 to 30.

The invention is based on the discovery that peptides comprising a hydrophobic core sequence (hereinafter "hydrophobic core" or "core") and pH-switchable hydrophilic N- and C-terminal sequences (hereinafter "hydrophilic termini" or "termini") are highly effective in enhancing membrane fusion. Peptides showing membrane fusogenic features were already described in the art (Hofmann, M.W. et al., PNAS, 2004, vol. 101, no. 41, 14776-14781). These state of the art peptides comprise a hydrophobic core and hydrophilic termini consisting of lysine residues. However, peptides having merely lysine residues at their termini do change their fusogenic activity within the pH range from 4 to 8, as encountered e.g. in endosomes. Thus, these prior art peptides are not usable for cell transfection of pH-sensitive or pH-labile therapeutically active substances via endocytosis. As a consequence, transfected material will be retained after cell entry in endosomal compartments and degraded under acidic conditions of the endosomes.

According to the invention peptides are provided, which are pH-switchable and which can be incorporated into lipid membranes, in particular spherical lipid bilayers, preferably liposomes. The term "pH-switchable" as used herein means that the inventive peptide displays different fusogenic activity depending on varying pH conditions. The conformation of inventive peptides depends, inter alia, on their charge, in particular on the charge of their hydrophilic termini. In general, peptide charge varies with the environmental pH. Protonation of histidine (H) occurs under acidic conditions, i.e. at pH < 7, leading to positively charged peptides. Under neutral or basic pH conditions, protonation of terminal histidine residue(s) in the inventive peptides does not occur. Without being bound to any theory, the inventive peptides comprising hydrophilic histidine termini may switch into another conformational state upon external pH change, thereby preferably altering its secondary structure conformation.

A preferred embodiment provides an inventive peptide comprising an amino acid sequence from at least 9 to 38 amino acids, wherein the amino acid sequence at its hydrophilic termini comprises at least one histidine residue at its N-terminus as well as at its C-terminus and from at least 6 to 30 essentially hydrophobic amino acids, preferably from at least 15, 16, 17, 18, 19 or 20 to 25 essentially hydrophobic amino acids, introduced between the hydrophilic termini.

A particularly preferred inventive peptide according to formula I above comprises two or three histidine residues at its hydrophilic N-terminus as well as at its hydrophilic C-terminus and 17, 18 or 19 essentially hydrophobic amino acids as hydrophilic peptide core. Other preferred peptides of the invention comprise hydrophilic termini comprising (i) one or two N-terminal histidine residues, and two or three C-terminal histidine residues or (ii) four or five N-terminal histidine residues, and three or four C-terminal histidine residues or (iii) two, three or four N-terminal histidine residue, and 5 or 7 C-terminal histidine residues etc.

All of these inventive peptides are typically characterized by a hydrophobic core of 10 to 30 essentially hydrophobic residues. As with any of the inventive peptides disclosed herein, more preferred peptides may comprise a hydrophobic core of 11 to 29, 12 to 28, 13 to 27, 14 to 26, 15 to 25, 16 to 24, 17 to 23, 18 to 22 or 19 to 21 amino acids.

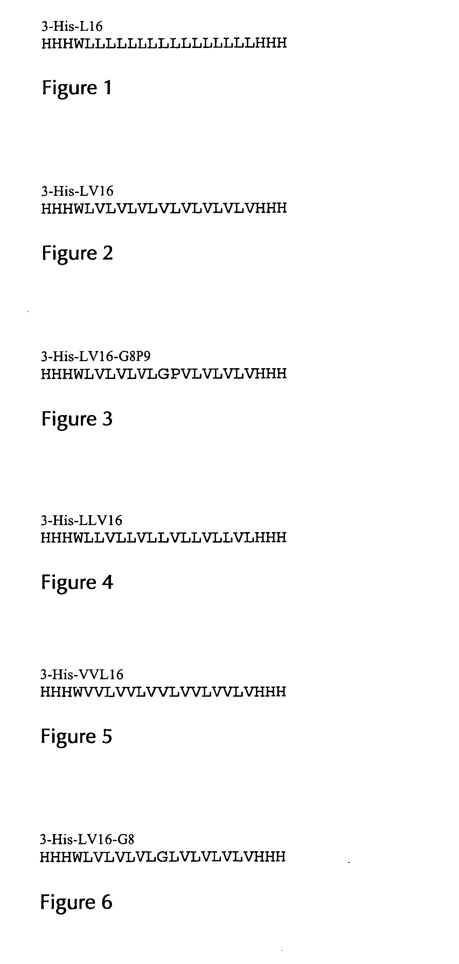

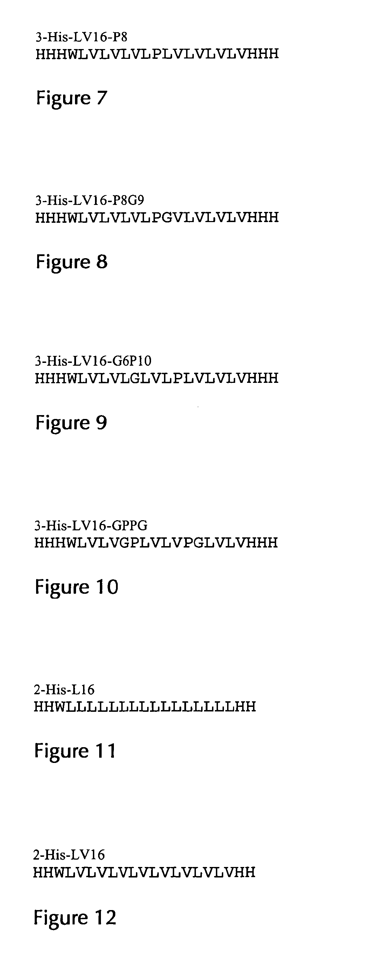

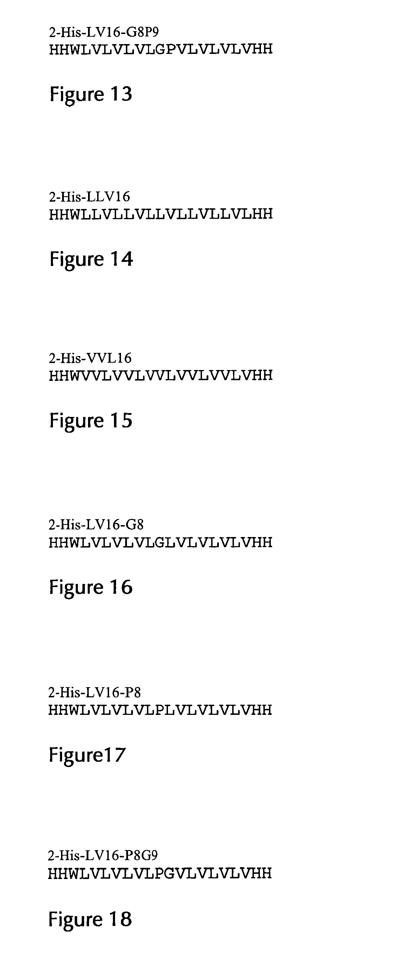

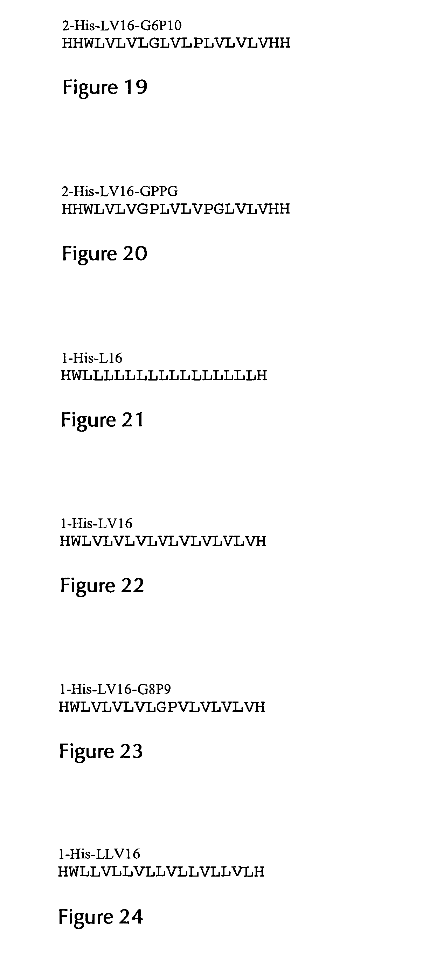

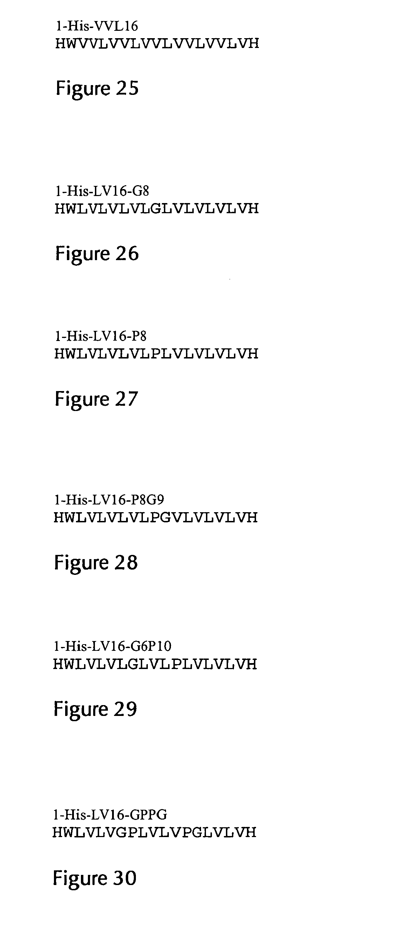

Other particularly preferred peptides of the invention comprise one of the amino acid sequences shown in figures 1 to 30.

The hydrophilic termini of inventive peptides comprise at least one histidine residue and optionally other essentially hydrophilic amino acids. The term "hydrophilic" amino acids or sequence refers to amino acids with polar amino acid side chain groups, such as histidine, forming energetically favorable interactions with water molecules rendering them readily soluble in water. The term "essentially hydrophilic amino acids" encompasses hydrophilic amino acid side chain groups, e.g., glutamine, glycine, asparagines, threonine, serine and tyrosine.

In either case, hydrophilic termini of inventive peptides comprise at least one histidine residue (component B of formula I) at either terminus. Histidine is a basic amino acid with a pKa value of 6. Under acidic conditions (pH < 7), protonation of terminal histidine residue(s) of the inventive peptides occurs. Protonation under acidic conditions leads to improved fusogenicity, which may be due to a conformational change of the inventive peptides. When inventive peptides being incorporated into lipid membranes are exposed to acidic conditions during cell transport (endocytosis, e.g. pH ≃ 5) protonation of the peptide termini destabilizes the structured lipid membrane and, thus, enhances fusion with other lipid membranes. Accordingly, inventive peptides are characterized by terminal histidine residues, which are charged under mildly acidic conditions as encountered e.g. in endosomes. Preferably, inventive peptides comprise two or more histidine residues at either hydrophilic terminus. According to a particularly preferred embodiment, the peptide of the invention comprises three histidine residues at its hydrophilic termini either in consecutive order or distributed among the 5 terminal residues of the peptide on either terminus. Nevertheless, hydrophilic N- or C-termini of an inventive peptide may differ, e.g., may be characterized by two histidine residues at its N-terminus and one or three histidine(s) at its C-terminus or vice versa, three histidine residues at its N-terminus and one or two histidine(s) at its C-terminus or vice versa etc.

Generally, the hydrophilic termini comprise 1 to 6 essentially hydrophilic amino acids, preferably 2 to 5 essentially hydrophilic amino acids.

The hydrophobic core comprises essentially hydrophobic amino acids, preferably selected from the group consisting of valine (V), leucine (L), isoleucine (I), proline (P), glycine (G), cysteine (C), tryptophane (W), alanine (A), phenylalanine (F) and methionine (M). The term "hydrophobic" amino acids or sequence according to the invention relates to amino acids with non-polar amino acid side chain groups that cannot form energetically favorable interactions with water molecules. The term "essentially hydrophobic amino acids" encompasses hydrophobic amino acids, e.g., alanine, valine, leucine, isoleucine, phenylalanine, proline and methionine. Preferably, the hydrophobic core of the inventive peptide does not comprise more than 15% of amino acids with (slightly) hydrophilic features. In other words, typically at least 85% , more preferably at least 90% even more preferably at least 95% of the amino acids of the hydrophobic core are "essentially hydrophobic" residues.

The hydrophobic core of an inventive peptide comprises from at least 6 to 30, preferably from at least 7 to 28, more preferably from at least 9 to 25, most preferably from at least 12 to 20, essentially hydrophobic amino acids placed between the hydrophilic termini. Particularly preferred inventive peptides comprise 15, 16 or 17 amino acids in their hydrophobic core.

Typically, the inventive peptide will contain in its hydrophobic core either a monotonous sequence of a hydrophobic amino acid, e.g. leucine, or a mixture of different hydrophobic amino acids. If different amino acids, preferably 2 to 6, more preferably 2 to 4, are present in the hydrophobic core, they may occur in any order, e.g. the different amino acids may be arranged clusterwise or may be distributed without any order scheme. If more than one amino acid type is present in the hydrophobic core, the amino acids shall preferably have different secondary structure forming propensities, e.g. a combination of leucine (strong ahelix propensity) and valine (strong β-sheet propensitiy) residues. In particular, any hydrophobic amino acid type combination in the hydrophobic core having at least one amino acid type with a helix propensity of at least 1.10 or any hydrophobic amino acid type combination having at least one amino acid type with a β-sheet forming propensity of at least 1.20 (according to table 1) is preferred. The amino acid type with a propensity of either character (strong helix former or strong β-sheet former) represents preferably at least 10%, more preferably at least 20% and even more preferably at least 30% and most preferably at least 40 % of the total number of amino acids in the hydrophobic core of the inventive peptide (depending on the number of different amino acid types occurring in the hydrophobic core). In a more preferred embodiment of the inventive peptide, the amino acid type combination in the hydrophobic core contains at least one amino acid type with a helix forming propensity of at least 1.10 and at least one amino acid type with a β-sheet forming propensity of at least 1.20. If the amino acid type combination is e.g. composed of two different amino acid types (with the amino acids distributed in any order), the helix forming amino acid type accounts typically for 20 to 80% of the amino acids of the hydrophobic core, more preferably 40 to 60%, while the remainder of the amino acids in the hydrophobic core are amino acids of the β-sheet forming amino acid type. If the amino acid type combination is e.g. composed of three or four different amino acid types (with the amino acids distributed in any order), the at least one helix forming amino acid type accounts typically for 20 to 60% of the amino acids of the hydrophobic core, more preferably 20 to 40% with at least 60 to 20% of amino acids, more preferably 40 to 20% , representing the β-sheet forming amino acid type, while the remainder of the amino acids in the hydrophobic core may belong to any hydrophobic amino acid type (either with or without secondary structure forming potential).

In another preferred embodiment, the hydrophobic core of inventive peptides forms a repeating pattern of more than one essentially hydrophobic amino acid type. Preferably, the repeating pattern is preferably formed by 2 to 4 essentially hydrophobic amino acids of different nature, i.e. different side chain moieties, even more preferably by a combination of amino acid types as indicated above with the preferred propensities.

Preferably, the core is formed by 2 (K and L) preferably alternating or clusterwise or distributed in any order, essentially hydrophobic amino acids. However, preferred patterns may be based on 3 or 4 different hydrophobic amino acids as well. General formula II, defining typical patterns of a hydrophobic core

((K)ₙ₁(L)ₙ₂(M)ₙ₃(N)ₙ₄)ₘ

wherein
n₁ or n₂ is an integer from 1 to 5,
n₃ or n₄ is an integer from 0 to 5 and
m is an integer from 1 to 15, provided that the core comprises from at least 6 to 30 amino acids,
considers alternative patterns with 2, 3 or 4 different essentially hydrophobic amino acids; wherein K, L, M and N may be any essentially hydrophobic amino acid. (variables K, L, M and N do not represent the single-letter-code for amino acids).

Accordingly, the number of repeats m depends on the length of the repeat unit. The larger the repeat unit, the smaller the number of repeats. The hydrophobic core may be composed by way of example of the following patterns: KLKLKLKL...; KKLLKKLLKKLL...; KKKLLLKKKLLL...;

Preferred repeat patterns are based on a proportion of 1/2 for either K and L, if n₃ and n₄ = 0, 1/3 for K, L and M, if n₄ = 0 or 1/4, if n₃ and n₄ ≠ 0. Examples of specific repeating patterns (1/3 for K, L and M, if n₄ = 0) are: KLMKLMKLMKLM...; KKLLMMKKLLMM...; KKKLLLMMMKKKLLLMMM.... Examples of specific repeating pattern (1/4, if n₃ and n₄ ≠ 0) are: KLMNKLMNKLMNKLMN...; KKLLMMNNKKLLMMNN...;

Alternatively, the hydrophobic core may represent repeating patterns without even distribution of the amino acids involved. Examples are KLLMMMKLLMMMKLLMMM....; KLMMKLMMKLMMKLMMKLMMKLMM....; KLLLLMKLLLLM.... etc.

KLLKLLKLLK; KKLKKLKKLKKL...; KKKLKKKLKKKL... etc. KKKLLLMMMNNKKKLLLMMMNNN....; KLLMMMNNNNKLLMMMNNNN....; KKLLMNKKLLMNKKLLMN....; KLMMNNKLMMNNKLMMNN.... etc.

Preferably, amino acids in the hydrophobic core of the inventive peptide can be arranged in palindromic order. For example following the pattern: ...KKLLMM/MMKKLL...;...KKKLLULLLKKK...;...KKLLMMNN/NNMMLLKK...;etc.

The (/) indicates the centre of the hydrophobic core. Palindromic sequences are defined to read the same irrespective of terminus which is selected as starting point. While the hydrophobic core may be arranged by any hydrophobic amino acid sequence, repeat patterns as disclosed herein form a specific embodiment of the present invention.

As described above the terminal hydrophilic residues of the inventive peptides fuse lipid membranes under acidic cellular conditions (into which an inventive peptide is incorporated). Thereby its fusogenic potential is enhanced. However, lipid membrane destabilization and its fusogenic potential may preferably be further increased by specific structural elements of the inventive peptide. These structural elements may be integrated into the hydrophobic core of inventive peptides. For example, preferred peptides contain at least one of amino acids proline and/or glycine. Their presence in the hydrophobic core destabilizes the helical secondary structure of the inventive peptide spanning the lipid bilayer. Upon pH change glycine and proline residues even further increase fusogenicity of the peptides. Fusogenic properties may be measured by any method known to the skilled person and by methods described herein.

According to the invention, peptides are preferred, which show a labile helical structure under acidic conditions, preferably at pH ≤ 6. Therefore, a preferred inventive peptide comprises at least one helical breaker, preferably at least one strong helical breaker, in its hydrophobic core. The term "helical breaker" relates to an amino acid comprised in the hydrophobic core, which strongly destabilizes the helical structure of an inventive peptide. Typically the 1 to 4, more preferably 1 to 3 helix breaking amino acids are distributed among the amino acids of the hydrophobic core. Particularly preferred helix breaking amino acids are proline and glycine. Preferably, the at least one helical breaker is/are arranged in the centre of the hydrophobic core (± 3 amino acids, forming the central portion). By way of an example, the central portion of the hydrophobic core of 20 amino acids in length spans amino acids 7 to 14, whereby the centre is defined by amino acids 10 and 11. Alternatively, at least two helix breaking amino acids are distributed over the hydrophobic core. Nevertheless, a symmetric arrangement of the helix breacking amino acids in the hydrophobic core is preferred. If more than one helical breaker is comprised in the core of the inventive peptide, they may be identical or different. A particularly preferred inventive peptide comprises 2 to 4 helical breakers chosen from proline and glycine in the central portion of the hydrophobic core. The amino acids proline and glycine may be arranged in any order in the central portion, preferably in consecutive order.

To determine and select suitable amino acids, which stabilize or destabilize secondary structure of the inventive peptide, one skilled in the art may select an appropriate hydrophobic core sequence according to their individual propensities. The term "propensity" defines the probability of an amino acid to exist in α-helical (also: helical), β-sheet (also: sheet) or coiled secondary structure. This propensity of an amino acid to form helical, sheet or coiled secondary structure can be calculated. Chou-Fasman (Biochemistry 13: 222-245,1974) developed a statistical method to classify propensities of amino acids to form secondary structures. This statistical method is based on the frequency to encounter each single amino acid in specific protein secondary structures as determined by X-ray diffraction. According to the Chou-Fasman method, so-called P-values (propensity values) are determined for each naturally occurring amino acid. By way of an example it may be said that the higher its P value as helix former, the more likely it is to observe an amino acid in a helical secondary structure. On the basis these P-values, amino acids can be divided into different groups as follows:
- strong helix former, helix former, weak helix former, helix indifferent, helix breaker and strong helix breaker and
- strong sheet former, sheet former, weak sheet former, sheet indifferent, sheet breaker and strong sheet breaker.

Details describing propensities of all 20 naturally occurring amino acids are given in following Table 1. Table 1 contains the P-value of each single amino acid residue indicating its propensity to occur in α-helical or β-sheet structures. The higher the P-value of an amino acid, the higher its secondary structure forming potential.

**Table 1:**

| Chou-Fasman Amino Acid Propensities | | | | |
|---|---|---|---|---|
| A.A. | Helix | | Sheet | |
| | Designation | P | Designation | P |
| Ala | H | 1.42 | i | 0.83 |
| Cys | i | 0.70 | h | 1.19 |
| Asp | l | 1.01 | B | 0.54 |
| Glu | H | 1.51 | B | 0.37 |
| Phe | h | 1.13 | h | 1.38 |
| Gly | B | 0.57 | b | 0.75 |
| His | I | 1.00 | h | 0.87 |
| Ile | h | 1.08 | H | 1.60 |
| Lys | h | 1.16 | b | 0.74 |
| Leu | H | 1.21 | h | 1.30 |
| Met | H | 1.45 | h | 1.05 |
| Asn | b | 0.67 | b | 0.89 |
| Pro | B | 0.57 | B | 0.55 |
| Gln | h | 1.11 | h | 1.10 |
| Arg | i | 0.98 | i | 0.93 |
| Ser | i | 0.77 | b | 0.75 |
| Thr | i | 0.83 | h | 1.19 |
| Val | h | 1.06 | H | 1.70 |
| Trp | h | 1.08 | h | 1.37 |
| Tyr | b | 0.69 | H | 1.47 |

| | | | | |
|---|---|---|---|---|
| A.A. indicates the amino acid in its triple code; P indicates the propensity for secondary structure; H indicates high propensity for secondary structure; h indicates intermediate propensity for secondary structure; I indicates weak propensity for secondary structure; i is inhibitory; B indicates a strong breaker of secondary structure; b indicates an intermediate breaker of secondary structure; | | | | |

As can be seen from Table 1, amino acids, which promote helical secondary structure include, e.g., alanine (Ala or A), aspartic acid (Asp or D), glutamatic acid (Glu or E), lysine (Lys or K) and methionine (Met or M). Amino acids, which promote sheet secondary structure include, e.g., cysteine (Cys or C), phenylalanine (Phe or F), isoleucine (Ile or I), threonine (Thr or T), valine (Val or V), tryptophane (Trp or W) and tyrosine (Tyr or Y). In particular, leucine (Leu or L) is an amino acid, which has the propensity to form helical as well as sheet secondary structure. Proline (Pro or P) and glycine (Gly or G) are indicated as (strong) breakers of secondary structure.

The mean P-value of all amino acids in the hydrophobic core of inventive peptides is preferably less than P = 1.2, more preferably less than P = 1.15, and even more preferably less than P = 1.1 for their helix forming potential. Preferably, the mean P-value of the hydrophobic core is between 0.9 and 1.25, more preferably between 1.0 and 1.2. Any residues (in any order) may preferably be employed for the hydrophobic core of an inventive peptide provided that the hydrophobic core maintains its hydrophobic character and the mean P-value for helix formation is preferably less than 1.25. For example, leucine has a P-value of 1.21, whereas valine has a P-value of 1.06 for helical structure. Therefore, a mean P-value of P = 1.135 (according to table 1) is calculated for a combination of leucine and valine each being represented proportionally in the hydrophobic core. Consequently, a preferred inventive peptide has a hydrophobic core consisting e.g. of leucine and valine residues in any order, preferably in a repeating pattern as disclosed above.

Preferably, inventive peptides comprise hydrophilic termini comprising at least one histidine residue and optionally other essentially hydrophilic amino acids at both termini (i.e. N-terminus and C-terminus) and a hydrophobic core comprising (i) amino acids that stabilize helical secondary structure of peptides, such as leucine, (ii) amino acids that stabilize sheet structure of peptides, such as valine, and (iii) amino acids that destabilize helical structure of peptides, such as proline and glycine.

Preferably, amino acids selected for the hydrophobic core have comparable P-values as either helix or sheet forming residues. Leucine, tryptophane, and phenylalanine are therefore preferred as amino acids for the hydrophobic core.

In another embodiment, the peptide according to the invention comprises a sequence of amino acids in its hydrophobic core exhibitions secondary structure propensities in the same order of magnitude, for both α-helix and sheet formation. Therefore, preferred inventive peptides comprise amino acids having similar P-values as helix and as sheet forming residues. Moreover, amino acids preferably forming coiled structure are also comprised in the hydrophobic core. These amino acids are indicated with "i" (inhibitory) in Table 1 above and encompass, in particular, cysteine, serine and threonine, or arginine. Consequently, amino acids forming helical or sheet secondary structure or coiled structure can be arranged in any suitable order and combination in the hydrophobic core of inventive peptides. However, different amino acids with the same propensity of helical, sheet and/or coiled secondary structures can also be combined to form an inventive peptide showing different propensities.

According to the invention, the inventive peptide can comprise naturally occurring amino acids (as described above) or non-naturally occurring amino acids.

In particular, the inventive peptide can comprise naturally occurring amino acids (L enantiomer) alternatively D enantiomers.

In a particularly preferred embodiment, the inventive peptide comprises at least one amino acid, which is usable for, e.g., fluorescent or spectrometric measurements. Preferred examples of these amino acids include tryptophane and tyrosine. This at least one fluorescent amino acid is preferably arranged between an hydrophilic terminus and the hydrophobic core of the inventive peptide (i.e. between components B and C of formula I above). However, this at least one amino acid may alternatively also be located at any other position within the hydrophobic core. Inventive peptides having fluorescent properties are particularly preferred for use in *in vitro* experiments applying, for example, FRET technique (Fluorescence Resonance Energy Transfer).

A peptide of the invention has fusogenic, membrane-permeabilizing, membrane-destabilizing, transfection, cellular transport, localization and/or receptor-ligand functions. Preferably, the peptide of the invention is able to alter transiently membrane structures upon changing pH conditions. A peptide of the invention has at least one of the aforementioned functions.

The production of a peptide according to the invention can be carried out following standard methods which are well known by a person skilled in the art (see e.g., Sambrook and Russell (2001) Molecular Cloning: A laboratory manual, Cold Spring Harbor Laboratory Press, Cold Spring Habor, NY). For example, peptides of the invention can be produced by recombinant methods. Recombinant methods are especially preferred, if a high yield is desired. A general method for the construction of any desired DNA sequence, and thus, deduced amino acid sequence is provided, e.g., in Brown J. et al. (1979), Methods in Enzymology, 68:109; Maniatis (2001), *supra.* Peptides of the invention can also be produced by *tert*-butoxycarbonyl-(boc)- or F-moc-synthesis as described in Examples below.

A preferred embodiment provides a peptide of the invention, which is incorporated in a spherical lipid bilayer. The term "spherical lipid bilayer" relates to a closed shell made up of lipid bilayers in the size of 40 nm to 10 µm in diameter encapsulating an aqueous core. "Lipids" according to the invention mean are amphiphilic compounds having a general structure composed of at least one hydrophobic and at least one hydrophilic section. Several hydrophobic sections may be connected through linkages containing at least one heteroatom that is different from carbon (e.g., nitrogen, oxygen, silicon, sulfur). A preferred spherical lipid bilayer of the invention is a liposome. A "liposome" according to the invention encompasses any type of liposome, such as cationic liposomes, unilamellar liposomes, multilamellar liposomes and vesicles. Preferably, a lipid constituent of a liposome of the invention is selected from the group consisting of phospholipids of synthetic or natural origin, for example phosphatidylserine (PS), phosphatidylethanolamine (PE), palmityloleyl-phosphatidylcholine (POPC), dioleyl-phosphatidylethanolamine (DOPE), dioleyl-phosphatidylserine (DOPS), stearyl-docosahexanyl-PE, stearyl-docosahexanyl-PS, lipofectine, lipofectamine, lipofectamine 2000 and colipids, such as POPE.

The production of a spherical lipid bilayer, in particular a liposome, according to the invention can be performed by any suitable standard method well known to a skilled person. For example, for the preparation of small unilamellar liposomes, the sonification in ice water is a usable method. A preferred method of the invention relates to the production of inventive liposomes by mechanical dispersion of lipids using ultrasonic treatment as described in Example 1.

A further preferred method is the mechanical dispersion of lipids for the preparation of spherical lipid bilayer, in particular liposomes, by extrusion. For extrusion, at first, MLVs (multilamellar vesicles) are prepared. These are forced with plunger syringes through a membrane of defined pore size (usually of polycarbonate). Lipid layers of MLVs are reorganized by the pressure applied, and liposomes having a homogeneous diameter in the region of the pore size of the membrane are formed. After 5 - 10 cycles, unilamellar liposomes are formed (LUVET = large unilamellar vesicles produced by extrusion techniques). The choice of the pore size of the particular membrane used allows preparation of liposomes of defined size. In contrast to high pressure homogenization, considerably less pressure is exerted on the vesicles, which makes this method gentler [2,5].

Another preferred method relates to the mechanical dispersion of lipids for the preparation of spherical lipid bilayer, in particular liposomes, by means of pressure homogenization. By high pressure, which is exerted, for example, by a so-called "French press" on MLVs prepared beforehand by rehydration with optional freeze drying, liposomes of smaller diameter and sharper size distribution are formed. By this procedure, fewer vesicle aggregates and more individual vesicles arise. A size minimum is reached at a diameter of about 20 nm. High curvature energies of the liposomes, which thereby occur, prevent a further reduction in the diameter. Nevertheless, as a consequence of applying very high pressures, degradation of liposomes by hydrolysis and oxidation of the lipids may occur [2,6].

A further preferred method relates to the mechanical dispersion of lipids for the preparation of spherical lipid bilayer, in particular liposomes, by freeze drying. In freeze drying, so-called multilamellar vesicles (MLV) are prepared beforehand by rehydrating a lipid film in aqueous buffer. These are subjected to freeze drying and then rehydrated again under gentle conditions. By exclusion of water, they allow a better contact between phospholipid layers and substances incorporated into the vesicles. The FRV (freeze-dried rehydration vesicles) thereby formed allow, in combination with an aforementioned extrusion procedure good results above all in respect of an efficient encapsulation of therapeutically active substances in liposomes [2,7,8].

Another preferred method is the mechanical dispersion of lipids for the preparation of spherical lipid bilayer, in particular liposomes, by freezing-thawing sonification. By freezing and subsequent thawing of SUVs prepared beforehand by sonification, reorganization and fusion of lipid layers of the liposomes take place. Their size thereby increases and the volume enclosed increases. By a subsequent brief ultrasonic treatment, relatively large amounts of substances can thus be "packed" into liposomes [2].

Furthermore, the dispersion of lipids in solvents is a preferred method for the production of spherical lipid bilayer, in particular liposomes. One approach relates to the injection from organic solvents- In this method, lipids are dissolved in an organic solvent (for example ethanol or ether) and injected rapidly with an excess of aqueous solution by means of a fine needle. By this operation, ethanol, the solvent for the lipids, is diluted suddenly and distributes these uniformly in the aqueous solution. SUVs are formed spontaneously. For this method an adequate mixing is important to avoid forming of lipid aggregates. A limiting factor of this method is the solubility of phospholipids [2,9].

Another preferred method for the production of spherical lipid bilayer, in particular liposomes is the dispersion of lipids in solvents by reverse phase evaporation (REV). In reverse phase evaporation, the material to be encapsulated, which is dissolved in water, is mixed with the lipids dissolved in an organic solvent. Inverse micelles are formed by this procedure. A water-in-oil emulsion is obtained by sonification. Viscous micelles which appear gelatinous are formed by slow evaporation of the solvent. Due to collapse of the gel state, the inverse micelles were transformed and an excess of phospholipids results. Lipid double layers are therefore formed around the remaining micelles. The liposomes thereby formed are called REV liposomes. Their size depends on the lipid and its solubility in the organic solvent, as well as the surface tension between the aqueous and organic phase and the contents of lipid and organic aqueous solvent [2,8,10].

Another preferred method for the production of spherical lipid bilayer, in particular liposomes relates to the dispersion of lipids in solvents by dissolving lipids by detergents. Colloidal solutions of phospholipids in aqueous buffers are obtained by the formation of mixed detergent/phospholipid micelles. By removal of the detergents, very homogeneous liposomes are formed. The detergents can be removed by dilution [11], dialysis [11], gel filtration [12] or adsorption [13]. By removal of the detergents unilamellar liposomes are formed spontaneously, which have a very homogeneous size distribution [2,8].

By incorporation of the peptide of the invention into a spherical lipid bilayer, in particular into a liposome, the peptide can stimulate, facilitate, effect or enhance the fusion of the liposome membrane and a biological membrane, preferably an endosomal membrane, due to its fusogenic and membrane active functions described above. A preferred embodiment refers to a complex comprising at least one peptide of the invention and a spherical lipid bilayer, preferably a liposome.

The incorporation of inventive peptides into a spherical lipid bilayer, in particular into a liposome, i.e. the preparation of an aforementioned peptide-spherical lipid bilayer-complex, can be carried out using any suitable standard method well known to a skilled person in the art. For example, peptides and liposomes of the invention can be mixed in an organic solvent followed by removing the organic solvent and subsequent suspending the dried mixture in water. Peptides and liposomes of the invention can also be mixed in an aqueous buffer via sonication or extrusion. Therefore, the peptides can be added to liposomes of the invention, whereby the peptides interact with lipid bilayers, whereas the specific amino acid sequence of the peptides is maintained (for more details see Examples 2, 3 below).

According to the invention, the peptide of the invention is not only usable to stimulate, facilitate, effect or enhance the above described membrane fusion, but also, by means of these mechanisms, to enhance the transfection efficiency of a therapeutically active substance, in particular a pH-sensitive or labile therapeutically active substance, into a cell or cell nuclei *in vitro* or *in vivo* in presence or absence of blood or serum. Preferably, the peptide and the therapeutically active substance are incorporated into a spherical lipid bilayer, in particular into a liposome. Thus, a preferred embodiment refers to a complex comprising at least one peptide of the invention, at least one therapeutically active agent and a spherical lipid bilayer, preferably a liposome. The preparation of such complex can be performed analogous to the preparation of a spherical peptide-lipid bilayer-complex, as described herein. However, a distinction has to be made as to whether the therapeutically active substance to be transported into the cell by means of liposomes is water-soluble or fat-soluble.

In general, the components peptide(s), therapeutically active substance(s) and spherical bilayer, especially liposome, are mixed in an aqueous buffer (if the therapeutically active substance(s) is/are water-soluble). The aqueous buffer is employed for resuspending the dried lipid/peptide mixture. In the case of the formation of liposomes, as described above, a portion of the substance to be transported is enclosed inside the liposomes. That proportion of substance to be transported, which has not been enclosed in liposomes is separated off using one of the following methods: dialysis, gel permeation chromatography, ultrafiltration, ultracentrifugation, extraction, free flow fractionation.

In the case of a fat-soluble therapeutically active, the components peptide(s), therapeutically active substance(s) and spherical bilayer, especially liposome, are mixed in an organic solvent, such as ethanol or ether, (if the therapeutically active substance(s) is/are fat-soluble) or a mixture of both. Suitable organic solvents can be polar or unpolar depending on the solubility characteristics of the fat-soluble therapeutically active substance(s) to be transported. Examples of usable organic solvents in the order polar → unpolar are ethanol, methanol, acetone, isopropanol, *tert*-butanol, dimethylsulfoxid (DMSO), pyridine, dichloromethane, trichloromethane, tetrahydrofuran (THF), dioxane, dimethylether, benzene, methylbenzene, tetrachloromethane, *n*-octane, *n*-hexane and cyclohexane.
Fat-soluble therapeutically active substances are preferably dissolved in a readily volatile organic solvent together with lipids and inventive peptides. The solvent is evaporated by passing over a stream of gas or under reduced pressure such that the remaining mixture of lipids, inventive peptides and the therapeutically active substance to be transported is distributed on the vessel wall over as wide an area and in as thin a layer as possible. This layer is suspended in aqueous buffer. Liposomes according to the invention can furthermore be prepared by the methods described above.

The term "therapeutically active substance" relates to a substance, which has potential to influence or alter physiological reaction(s) and/or function(s), in particular cellular process(es). A cellular process in this context is a process associated with a cell before the transfection of a therapeutically active substance. For example, the transfection of a therapeutically active substance of the invention can effect, stimulate or inhibit the cellular production of a substance/molecule, such as a protein. Therapeutically active substances of the invention can be hydrophilic, hydrophobic or amphiphilic. Examples of therapeutically active substances of the invention are pharmaceuticals, drugs (e.g. anti-cancer drugs, like paclitaxel, doxorubicin, daunomycin, anthracyclines, dactinomycin, bleomycin, adriamycin, mithramycin, daunorubicin and idarubicin, vincristine, carboplatin and cisplatin, oxaliplatin, thioguanine, alkylating agents, e.g. nitrogen mustards; ethylenimes; alkylsulfonates; triazenes; piperazines; and nitrosureas; cyclosporamide, (anionic) therapeutic agents and antigens, hydrophobic substances, such as hydrophobic organic molecules of low molecular weight, steroids, terpenes, triglycerides, prostagladines, anthrachinones, and hydrophilic substances, such as hydrophilic organic molecules of low molecular weight, functionalized heterocycles, sugars, cytokines, proteins, e.g., erythropoietin, viral coat proteins and enzymes, enzyme inhibitors, hormones, e.g., growth hormones, such as human growth hormone (hGH), polypeptides, peptides, oligonucleotides, nucleic acids and expression vectors containing said nucleic acids. Nucleic acids of the invention comprises any nucleic acid, for example, DNA, cDNA, anti-sense DNA, RNA, mRNA, tRNA, snRNA, siRNA, anti-sense RNA, ribozymes and recombinant nucleic acids, without size limits from any source comprising natural and non-natural bases. Nucleic acids of the invention can have a variety of biological functions. They can encode proteins, comprise regulatory regions, function as inhibitors of gene or RNA expression, function as inhibitors of proteins, function to inhibit cell growth or kill cells, catalyze reactions or function in a diagnostic or other analytical assay.

The term "transfection" means the delivery and introduction of a therapeutically active substance of the invention into a cell or into its organelles, whereby the therapeutically active substance deploys its function.

A "cell" according to the invention relates to any suitable cell, especially an eukaryotic cell, preferably a mammalian cell, more preferred a human cell, a murine cell, a bovine cell, equine cell, a feline cell and an ovine cell. The transfection of a therapeutically substance of the invention can be effected, for example, into a muscle cell (e.g., skeletal, heart, respiratory, striated and non-striated), liver cell (e.g., hepatocytes), spleen cell, immune cell, gastrointestinal cell, cell of the nervous system (e.g., neurons, glial, and microglial), skin cell (e.g., dermis and epidermis), joint and synovial cell, tumor cell, kidney cell, cell of the immune system (e.g., dendritic cell, T cell, B cell, antigen-presenting cell, macrophages), exocrine cell (e.g., pancreas cell, salivary glands), prostate cell, adrenal gland, thyroid gland, retinal cell and respiratory cell (e.g., cell of lung, nose and respiratory tract).

Transfection procedures of the invention can be applied to cells *in vitro* or *in vivo.* The transfection efficiency of a therapeutically active substance is "enhanced" by a peptide according to the invention when an increase of at least about 5 %, preferably about 10 %, more preferably about 20 % in transfection efficiency is shown compared to the efficiency of a transfection of the corresponding therapeutically active substance under the same conditions but without the peptide of the invention.

In a preferred embodiment, the invention provides a method for transfecting a cell comprising the following steps:
a. providing a complex that contains a peptide, a therapeutically active substance and a spherical lipid bilayer of the invention and
b. contacting a cell with the complex from step a.

A complex according to step a. of the method can be provided by adding a peptide and a therapeutically active substance of the invention to a spherical lipid bilayer of the invention.

Contacting a cell with the complex of step a. of the method can be carried out *in vitro* or *in vivo.* For example, a cell of a cell or tissue culture can be contacted with the complex *in vitro* by adding the complex to the tissue culture. If desired, these transfected cells can be introduce into a mammal to be treated by any suitable standard method for the introduction of cells into an organism. The contact *in vivo,* and in general the administration of peptides and complexes of the invention, can be performed by any suitable route, preferably by subcutaneous injection or injection into a vein. The route of administration and the dosage of peptides, complexes and therapeutically active compounds will depend on various conditions, e.g., the desired purpose, the nature of therapeutically active compound, the organism to be treated. One of ordinary skilled in the art, in particular a physician, based on knowledge generally available to the art including the present disclosure, can administer the peptides, therapeutically active compounds complexes and cells containing said peptides, therapeutically active compounds and/or complexes with any delivery system without the expense of undue experimentation.

It will be apparent to a skilled person in the art that alternative methods, reagents, techniques other than those specially detailed herein can be used or readily adapted to produce inventive peptides, spherical lipid bilayers or complexes or to effect described inventive membrane fusion or cell transfection.

As mentioned above, the peptide of the invention influences membrane fusion and cell transfection. Therefore, a preferred embodiment relates to the use of a peptide or a complex of at least one peptide, at least one therapeutically active compound and a spherical lipid bilayer of the invention for effecting and/or enhancing of cell transfection efficiency.

Another preferred embodiment refers to the use of an inventive peptide or an inventive complex for stimulating and/or effecting a pH-switchable membrane fusion between the membrane of a spherical lipid bilayer and the membrane of a cell. For this use, the complex can comprise at least one peptide and a spherical lipid bilayer and optionally at least one therapeutically active compound depending of desired purposes.

The peptides and complexes of the invention are for example useful in gene therapy, vaccination, immunization, in vitro transfection of mammalian cells, delivery of therapeutic compounds into cells and tissues and for generating an immune response.

Another preferred embodiment of the invention relates to a kit comprising at least one peptide, at least one therapeutically active substance and a spherical lipid bilayer, preferably a liposome of the invention.

In summary, according to the invention, an sustained and efficient transfection of therapeutically active substances, in particular pH-sensitive or labile therapeutically active substance into cells by enhanced membrane fusion under acidic pH conditions effected by the peptide of the invention is demonstrated. Moreover, this transfection and membrane fusion can be controlled by the peptide of the invention not only by varying pH values but also by a specific hydrophobic core of the peptide.

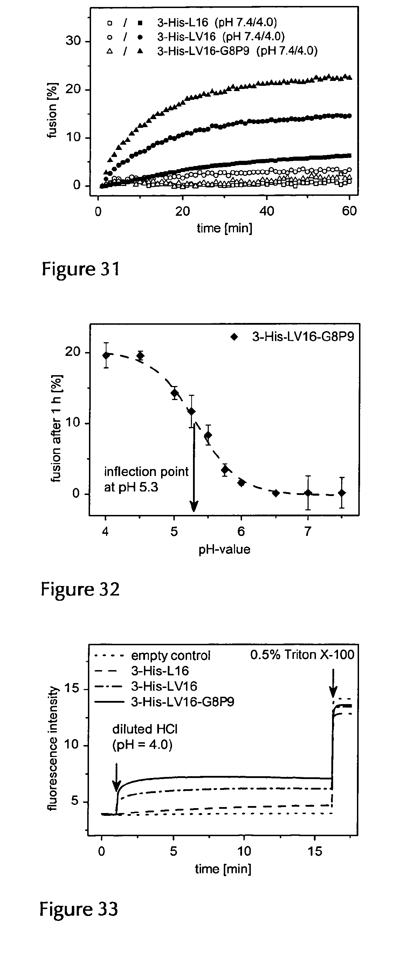

To investigate the membrane fusogenic potential of the peptides of the invention, the inventors have incorporated them in liposomes. The determination of fusion kinetics was performed using fluorescence resonance energy transfer (FRET) technique, as described in the Examples. For this purpose, liposomes usable for FRET measurements were prepared. Therefore, fluorescent dyes, e.g., N-(7-nitorbenz-2-oxa-1,3-diazol-4-yl)-dihexadecanylphosphatidylethanolamine (NBD-PE) or Lissamine^{™}rhodamine B-dihexadecanylphosphatidylethanolamine (Rh-PE), were incorporated into liposomes of the invention. In this context, it is mentionable that FRET technique requires the preparation of different liposomes, namely a donor liposome and an acceptor liposome carrying different fluorescent dyes. The resulting destabilization and membrane fusion efficiency of liposomes in which the peptides of the invention were incorporated, measured by FRET, (see Figures 31 to 33) was promoted by protonation of N- and C-terminal histidine residues. However, because of the low pKa value of histidine of pKa 6, this protonation especially occurs at acidic pH values, preferably in the range of pH 4 to 6, more preferably in the range of pH 4.5 to pH 5.5, most preferably in the range of pH 5 to pH 5.3. It is known that the acidic pH conditions in endosomes relate to a pH value of about 5. Thus, in contrast to known fusogenic peptides in the art, the peptides of the invention are highly usable for transfecting therapeutically active substances into cells, whereby the substances are taken up into endosomes by endocytosis. Moreover, because of the acidic pH specificity of inventive peptides, an early and unspecific membrane fusion with the plasma membrane (under pH conditions of about pH 7.5) is avoided. Thus, the therapeutically substance will be only released, when the fusion of liposome membrane and inner membrane of (early or late) endosomes occurred. Moreover, the inventors have demonstrated an enforcing influence of the specific hydrophobic core on membrane fusion. This influence results from the specific formation of different amino acids with different features, e.g., amino acids that stabilize helical structure of inventive peptides, amino acids that stabilize sheet structure of inventive peptides and amino acids that destabilize helical structure of inventive peptides.

The following figures and examples are intended to illustrate the invention without limiting the scope of the invention as claimed in the claims.

### Figures

**Figure 1** shows the amino acid sequence of the peptide 3-His-L16.
**Figure 2** shows the amino acid sequence of the peptide 3-His-LV16.
**Figure 3** shows the amino acid sequence of the peptide 3-His-LV16-G8P9.
**Figure 4** shows the amino acid sequence of the peptide 3-His-LLV16.
**Figure 5** shows the amino acid sequence of the peptide 3-His-VVL1 6.
**Figure 6** shows the amino acid sequence of the peptide 3-His-LV16-G8.
**Figure 7** shows the amino acid sequence of the peptide 3-His-LV16-P8.
**Figure 8** shows the amino acid sequence of the peptide 3-His-LV16-P8G9.
**Figure 9** shows the amino acid sequence of the peptide 3-His-LV16-G6P10.
**Figure 10** shows the amino acid sequence of the peptide 3-His-LV16-GPPG.
**Figure 11** shows the amino acid sequence of the peptide 2-His-L16.
**Figure 12** shows the amino acid sequence of the peptide 2-His-LV16.
**Figure 13** shows the amino acid sequence of the peptide 2-His-LV16-G8P9.
**Figure 14** shows the amino acid sequence of the peptide 2-His-LLV16.
**Figure 15** shows the amino acid sequence of the peptide 2-His-VVL16.
**Figure 16** shows the amino acid sequence of the peptide 2-His-LV16-G8.
**Figure 17** shows the amino acid sequence of the peptide 2-His-LV16-P8.
**Figure 18** shows the amino acid sequence of the peptide 2-His-LV16-P8G9.
**Figure 19** shows the amino acid sequence of the peptide 2-His-LV16-G6P10.
**Figure 20** shows the amino acid sequence of the peptide 2-His-LV16-GPPG.
**Figure 21** shows the amino acid sequence of the peptide 1-His-L16.
**Figure 22** shows the amino acid sequence of the peptide 1-His-LV16.
**Figure 23** shows the amino acid sequence of the peptide 1-His-LV16-G8P9.
**Figure 24** shows the amino acid sequence of the peptide 1-His-LLV16.
**Figure 25** shows the amino acid sequence of the peptide 1-His-VVL16.
**Figure 26** shows the amino acid sequence of the peptide 1-His-LV16-G8.
**Figure 27** shows the amino acid sequence of the peptide 1-His-LV16-P8.
**Figure 28** shows the amino acid sequence of the peptide 1- His-LV16-P8G9.
**Figure 29** shows the amino acid sequence of the peptide 1-His-LV16-G6P10.
**Figure 30** shows the amino acid sequence of the peptide 1-His-LV16-GPPG.
**Figure 31** shows the fusogenic behaviour of the peptides 3-His-L16, 3-His-LV16 and 3-His-LV16-G8P9 of the invention at neutral pH (pH = 7.4; framed symbols) and acidic pH (pH = 4 filled out symbols). The increase in fusion (corresponding to increase in kinetics) is plotted over time. As can be seen, almost no fusion occurred at pH 7.4. In contrast, at pH 4 fusion was determined. Apart from the pH conditions, the level of fusion was also dependent on the hydrophobic core. 3-His-L16 (square symbols) shows slightly increasing kinetics, 3-His-LV16 shows a significant increase and 3-His-LV16-G8P9 shows the strongest increase. The peptide/lipide (P/L) ratio was 0.005 in each case. **Figure 31** shows kinetic mean values from three independent measurements in each case. Spontaneous fusions of liposomes without peptides were measured in parallel and were subtracted.
**Figure 32** shows the fusion of 3-His-LV16-G8P9 (which shows the strongest fusion increase at pH 4 in **Figure 31**) at various pH values whereby reconstitution was carried out at indicated pH values. The fusion values after 1 hour are plotted vs. respective pH values. The results show a sigmoidial curve (dashed line) corresponding to the His-titration-curve showing an inflection point at pH 5.3. The results confirmed again that fusion can be controlled via the level of protonation of histidine residues of the peptides of the invention.
**Figure 33** shows liposomes produced at pH 6.5 and the time-dependent progress of fluorescence, which was measured as an indicator for liposome fusion. At t = 1 min. (arrow left side) the pH was abruptly decreased to pH 4 by addition of diluted hydrochloric acid. At t = 16 min, liposomes were lysed by addition of triton X-100 (at end concentration of 0.5 %), and thus, the maximal fluorescent value was obtained (corresponding to 100 % fusion) (arrow right side). It can be seen that liposomes without peptides (blank liposomes; dotted line) show no fusion after acidification. In contrast, liposomes containing peptides show fusion, the level of which is dependent on the hydrophobic core (analogous to Figure 32).

The results of Figures 31 to 33 show that peptides of the invention are usable to influence the fusion of liposomes containing them. The degree of fusion can be controlled by external pH conditions. Also, the core sequence of the inventive peptide may have some influence on the fusogenic activity.

### Examples

### Materials

The peptides used were prepared by tert-butoxycarbonyl (Boc) synthesis [1] (PSL, Heidelberg). They were obtained as lyophilisate. Since they are TFA (trifluoroacetate) salts from the synthesis, after being obtained they were suspended in milliQ-H₂O and neutralized by means of dilute ammonia solution. The pH was checked by means of pH paper and increased from about 3 to 7.5. The peptides were then lyophilised again (overnight). To minimize decomposition, from then on they were kept in a freezer at -80 °C.

Phosphatidylcholine (PC) was purified from chicken egg yolk (Serva) by means of column chromatography [2]. For this, 62.5 g anhydrous aluminium oxide (activity level I) were suspended in 75 ml CHCl₃ and the solution was introduced into a chromatography column (diameter approx. 2 cm, height approx. 40 cm). After the Al₂O₃ had settled, 2.5 g egg-PC, dissolved in 50 ml CHCl₃, were introduced on to the column. Thereafter, the column was washed with 40 ml CHCl₃ at a flow rate of approx. 10 ml/min. Elution was carried out slowly by CHCl₃/MeOH 90:10 (v/v). The first 50 ml were discarded. Thereafter, fractions of 15 ml each were collected in CHCl₃-stable 50 ml Falcon tubes. The purity of PC in the fractions collected was checked by thin layer chromatography (TLC) [2]. For this, in each case about 5 µl were applied to fluorescence-labelled TLC plates of silica gel 60 (Alugram SIL G/UV₂₅₄). The mobile phase was CHCl₃/MeOH/H₂O 65:25:4 (v/v/v). Detection of the lipids on the plate was performed either by means of UV light, or they were placed in an iodine chamber for about 5 min, whereupon brown spots were formed.

The purity of PC in the fractions was determined in comparison with a standard with the aid of the retention factors (R_{f}). The R_{f} values were: PC = 0.25; PE = 0.5; cholesterol = 0.88. The fractions with pure PC were combined in a flask, which had been weighed empty beforehand, and the solvent was removed on a rotary evaporator (about 150 mbar). After drying in vacuo (20 mbar) for about 1 hour, the weight of the PC was determined. The lipid was dissolved in CHCl₃ to 10 mg/ml and divided into aliquots in small brown glass bottles.

Phosphatidylserine (PS) and phosphatidylethanolamine (PE), purified from brain, and the synthetic lipids palmityloleyl-phosphatidylcholine (POPC), dioleyl-phosphatidylethanolamine (DOPE), dioleyl-phosphatidylserine (DOPS), stearyl-docosahexanyl-PE and -PS were purchased from Avanti (Avanti Polar Lipids, USA). They were also dissolved in CHCl₃ to 10 mg/ml and diluted, respectively. The fluorescence dyes N-(7-nitrobenz-2-oxa-1,3-diazol-4-yl)-dihexadecanylphosphatidylethanolamine (NBD-PE) and Lissamine^{™} rhodamine B-dihexadecanylphosphatidylethanolamine (Rh-PE) were purchased as solids from Molecular Probes. After dissolving in CHCl₃ to 1 mg/ml, they were divided into aliquots in small vessels. Since relatively inaccurate values were obtained during weighing on an analytical balance, the concentrations of the two dyes were determined by recording a UV absorption spectrum. The concentration was calculated for NBD-PE by absorption at 460 nm (E = 21,000 M⁻¹cm⁻¹) and for Rh-PE at 530 nm (E = 75,000 M⁻¹cm⁻¹). After use, all the lipids were always covered with a layer of nitrogen and, after closing airtight with Parafilm, stored in a freezer at-20 °C.

All the other chemicals used were analytical grade. All the buffers were prepared in milliQ water and the stock solutions were filtered through a circular filter (pore size 0.23 µm) by means of a disposable syringe.

White, non-binding 96-well plates from Corning (article no. 3604, Conring Inc., USA) with transparent bases were used for measurement of the fluorescence kinetics. Before being used for the first time and after each use, these were first cleaned with the detergent Triton X-100, then washed with 96 % ethanol and finally rinsed with dist. water. After drying overnight at room temperature, the plates showed constant fusion results, and could be re-used about 20 times. For comparison, transparent 96-well microtitre plates from Sarstedt (nr. 82.1581; Sarstedt, USA) were also used. After the usual pre-cleaning (see above), these plates gave identical results.

### Equipment

The quantification of the peptides was carried out on an Ultrospec 3100pro UV/Vis spectrometer (Amersham Bioscience, USA) using a quartz microcell (volume 100 µl). The data were printed out by means of a thermal printer, model DPU-414 (Seiko Instruments Inc., Japan).

A thermomixer (Eppendorf, Hamburg, DE) was used for rehydrating the lipid films. Sonification of the lipid suspension took place in a Branson sonifier. In this, the ultrasonic generator (type 102C) excited the cup resonator by means of a 450 power supply unit (Branson, USA). The apparatus was cooled constantly with ice-water during operation.

The fusion kinetics were recorded on a BMG FLUOstar (BMG LabTechnologies, USA) by measurement of the fluorescence in 96-well plates. The evaluation was performed by the FLUOstar Galaxy Version 4.21 computer software and the Microsoft Excel table calculation program.

Fusion kinetics of liposomes with histidine peptides were recorded in fluorescence cuvettes of quartz glass (thickness: 1 cm) on an RF-1501 fluorescence spectrometer (Shimadzu, Japan). The evaluation was performed by the Hyper-RF software. The quantification of tryptophan (for details, see below) was also carried out on this instrument.

The ultracentrifugation was carried out in centrifuge tubes of polyallomer in an SW60-Ti rotor in an LE-80K ultracentrifuge (Beckman-Coulter, USA).

Detection of *p*-cresol by means of HPLC took place with a Waters system type Alliance (mod. 2695) with a photodiode array (PDA) detector (mod. 996) on an analytical Vydac C₄ column (214TP54, 250 x 4.6 mm, GraceVydac, USA). The evaluation was performed by means of Millenium32 software (Waters, USA). All mass spectrometry measurements were carried out on a Waters QTof-Ultima electrospray ionization (ESI) mass spectrometer. The data were evaluated by Masslynx 4.0 software (Waters, UK).
The lyophilization was carried out in an apparatus of the Delta 1-20KD type (Christ, Osterode, DE).

### Example 1: Peptide quantification

The peptides were stored at -80 °C in the form of their lyophilisates. For dissolving, they were first temperature-conditioned to room temperature in the closed vessel for at least 30 min. They were then weighed into Eppendorf reaction vessels (2 ml) on an analytical balance and dissolved in TFE (trifluoroethanol) to about 2 mg/ml. Undissolved peptide was removed by centrifugation (13,000 rpm, 4 °C, 10 min). Since because of their high hygroscopicity the value determined on the balance was too inaccurate and the amount of undissolved peptide was unknown, the concentration of the peptide in the supernatant was determined by means of the absorption of tryptophan. The measurement was carried out in TFE/DMSO (dimethylsulfoxide) 1:1 (v/v) and an absorption spectrum was recorded against TFE/DMSO 1:1 (v/v) in the range of from 220 to 330 nm. The concentration of the peptide was calculated (about 400 µM) on the basis of the Lambert-Beer law with the aid of the absorption maximum at 282 nm (= 5,600 M⁻¹·cm⁻¹).
Alternatively (for SPE), peptide was dissolved in acetonitrile (AcN)/H₂O 20:80 (v/v) and measured against AcN/H₂O 80:20 (v/v). The concentrations determined, the date and the name of the peptide were noted on the reaction vessel. Dissolved peptides were stored at - 20°C.

### Example 2: Preparation of liposomes and measurement of the fusion kinetics

The fusion kinetics were determined by measurement of the fluorescence resonance energy transfer (FRET) from NBD-PE to Rh-PE [3]. Three species of liposomes were prepared for this: i. liposomes without peptide, ii. liposomes with peptide and iii. donor liposomes with the dyes NBD-PE and Rh-PE.

The lipids PC, PE and PS dissolved in CHCl₃ (conc. each 10 mg/ml) were mixed in the ratio 3:1:1 (v/v/v) in a 2 ml reaction vessel. 600 µl liposome suspension were prepared as standard (volume of the buffer used later on rehydration). 90 µl PC, 30 µl PE and 30 µl PS were used for this, which on the basis of the molecular weight of the lipids (approx. 800 g/mol) corresponds to an amount of lipid of 1.87 µmol. In 600 µl buffer, the lipid concentration was 3.1 mM or 2.5 mg/ml. Peptides were incorporated into the liposomes in molar peptide/lipid (P/L) ratios of nominal 0.0025 to 0.01. The amount of peptide dissolved in TFE required for this was calculated from the previously determined concentration and the required P/L ratio. For 1 mol% peptide (corresponding to 0.0187 µmol or 18.7 nmol), 46.8 µl of a peptide solution of a concentration of 400 µM were accordingly used. For the preparation of donor liposomes, in each case 0.8 mol% (corresponds to 15 nmol) of the dyes NBD-PE (M_{R} = 956.3 g/mol) and Rh-PE (M_{R} = 1,333.8 g/mol) were added. Since the concentration of the two dyes was in each case 1 mg/ml, 14 µl NBD-PE and 20 µl Rh-PE had to be used for this. For the preparation of liposomes with DHA lipids, either only PE, only PS or PE and PS were replaced by synthetic lipids, wherein the lipid composition PC/PE/PS 6:2:2 (w/w/w) was retained. Thus, for example, the 20 % natural PE from brain was replaced by 20 % SOPE (corresponding to 0 % DHA). 5, 10 and 20 % S(DHA)PE were then used, and supplemented with SOPE to 20 %. After pipetting together the lipids / peptides / dyes, the solutions were homogenized thoroughly (Vortexer). By rotating the vessel held on a slant, a thin film of liquid was produced by means of a gentle stream of nitrogen. Complete drying of the film to remove CHCl₃ and TFE was carried out in vacuo overnight. The lipid film was rehydrated in aqueous buffer. For this, in the normal case 600 µl "fusion buffer" (150 mM NaCl / 20 mM Tris-HCl (pH 7.4) / 5 mM DTT / 0.1 mM EDTA) were added and the vessel was covered with a layer of nitrogen. The formation of multilamellar liposomes took place by shaking (1,400 rpm) at 37 °C for 1 h. Thereafter, the samples were cooled constantly in an icebath.

Small unilamellar liposomes (SUV = small unilamellar vesicles) were prepared by sonification in ice-water. For this, about 10 min before the procedure the cup resonator on the sonifier was cooled with ice-water. The samples were hung into the cup either individually or in threes, by means of the appropriate attachment, and fixed at the top against being pushed out using a support clamp. Sonification was carried out for 8 min at 80 % amplitude (output 35-38 %).

Aggregates and larger liposomes were removed by centrifugation (13,000 rpm / 4 °C / 20 min). Bleaching of NBD in donor liposomes for measurement of the mixing of the inner lipid layer was carried out by 20 mM dithionite (DTN; M_{R} = 174.1) on ice for 30 min. 100 µl of the dye-labelled liposomes were mixed thoroughly with 10 µl of freshly prepared dithionite solution (200 mM or 34.6 mg/ml) and stored in the dark on ice. Column chromatography ("spin column") was carried out to remove the DTN in the solution. Sephadex G50 fine (Amersham Bioscience, Sweden) was dissolved in fusion buffer (150 mM NaCl / 20 mM Tris-HCl (pH 7.4) / 0.1 mM EDTA) and left to swell overnight. The columns were prepared from 2 ml disposable syringes, which were inserted into 15 ml Falcon tubes. An antibiotic test platelet was first introduced into the column, and this was then filled to the top edge with the Sephadex G50 solution (after suspending). Centrifugation was carried out for 7 min at 1,000 rpm and 4 °C. After filling the column again, centrifugation was carried out for 3 min at 1,000 rpm and 4 °C. After replacement of the 15 ml Falcon tube, the sample was applied (110 µl) and centrifugation was carried out for 3 min (1,000 rpm, 4 °C). By this way, bleached liposomes were obtained in the runnings.

Donor liposomes (iii.), with and without DTN treatment, were mixed with the other liposomes (i. or ii.) in the ratio 1:4 on ice and the mixture was pipetted into the 96-well plates. The drift control here comprised one part of donor liposomes and 4 parts of buffer. This monitored the decomposition of the dye NBD, the adsorption of the liposomes on the vessel wall and the electronic drift of the instrument during the fluorescence measurement in the kinetics.
After temperature-conditioning of the plate at 37 °C (water-bath) for two minutes, the measurement in the reading instrument (BMG FluoSTAR) was started immediately. The fluorescence of NBD at 530 nm (excitation 460 nm) was recorded at intervals of 1 min (total duration 60 minutes). Thereafter, the liposomes were destroyed by adding in the detergent Triton X-100 (final concentration 0.5 %) and the maximum fluorescence was measured. By table calculation (Microsoft Excel), the kinetics were calibrated to 100 % from the difference from the initial value.

To characterize the influence of AmpOX, this was weighed on an analytical balance and dissolved in DMSO. After dilution of the resulting stock solution, corresponding amounts were added to the lipid/peptide mixtures before the drying. The liposomes were reconstituted as described above.

### Example 3: Alternatives in the preparation of peptide-containing liposomes

Alternatively to incorporation of the peptides into the liposome membrane by simultaneous drying of a lipid/peptide solution, the peptides can also be added from an organic solvent to previously prepared liposomes (without peptide). In this case, the peptides interact with the lipid double layers and the sequence specificity of the peptide-induced fusion is retained.

The use of other mixture ratios of the lipids (the phospholipids PC, PS and PE are used as standard) and the presence of other lipids, such as sphingomyelin and cholesterol, also allow a sequence-dependent peptide-induced liposome fusion.

### Example 4: Determination of the P/L ratio

The peptide/lipid (P/L) ratio in liposomes with peptide (ii.) was determined as described in [4], which is incorporated herein in its entirety by reference. For this, non-bound peptide was first removed from an aliquot of the samples, with which the fusion kinetic had previously been recorded, by ultracentrifugation (UC). 300 µl of the particular sample were mixed with 700 µl 60 % sucrose (w/v) at the bottom of a centrifuge tube (polyallomer; 11 x 60 mm; Beckman, USA) and covered with a layer of 2.5 ml 30 % sucrose (w/v) by cautious dropwise addition from a 10 ml syringe without a plunger together with a long cannula. After covering with a layer of a further 0.5 ml buffer (150 mM NaCl; 20 mM Tris (pH 7.4); 0.1 mM EDTA) by means of a 1,000 µl Eppendorf plunger pipette, the tubes were placed in the centrifuge cups. These were tared to ± 1 mg on an analytical balance. The UC was carried out in the SW60Ti rotor at 56,000 rpm (322,000 x*g*) for 20 h at 20 °C. Thereafter, fractions of 1 ml each were removed from the bottom upwards with a 1 ml syringe and a long needle. To avoid mixing of the fractions, the needle was always immersed cautiously at the edge of the solution, and pulled out again cautiously after the fraction had been taken off. The liposomes were contained in the upper fraction No. 4, non-bound peptide remained in fraction No. 1. Fractions Nos. 2 and 3 were discarded.

The peptide from fraction No. 4 incorporated in liposomes was quantified by measurement of the tryptophan fluorescence. 100 µl of the fraction were diluted in 800 µl buffer (150 mM NaCl, 20 mM Tris (pH 7.4), 0.1 mM EDTA) and 100 µl 10 % SDS (w/v; final concentration 1 %) in the fluorescence cuvette. With constant stirring, an emission spectrum between 220 and 400 nm was recorded on the Shimadzu RF-1501 fluorescence spectrometer (excitation 290 nm). The maximum at 320-323 nm was read off. A blank value (liposomes without peptide; i.) was first measured and the fluorescence value A was thereby obtained. Measurement of liposomes with peptide gave the value B. The peptide samples were increased in concentration twice (B1 and B2) with peptide of known concentration (internal standard, dissolved in TFE). From their difference (B2-B1) and the value B and the blank value A, the concentration of the peptide was calculated from the stated amount of internal standard.

The lipids were quantified by formation of a molybdophosphate complex of the contained phosphate group. A duplicate determination was carried out in this context. In each case 75 µl of fraction No. 4 from the UC were first divided into aliquots in test tubes and broken down with 400 µl 70 % perchloric acid at 200 °C for 1.5 hours on a heating block. The determination was carried out in parallel with phosphate standard solutions (0, 20, 40, 60, 80 and 100 mmol). After cooling of the clear solutions, in each case 2.4 ml of a 0.44 % (w/v) solution of hexa-ammonium heptamolybdate 4-hydrate and 2.4 ml of a 2.5 % (w/v) solution of Fiske-Subbarow reducing reagent were pipetted in and the test tubes were mixed thoroughly (Vortexer, 1,600 rpm). After boiling for 10 min in boiling water, the test tubes were cooled in ice-water. One millilitre of the solutions was transferred into cuvettes of plastic. The absorption at 820 nm was measured on the UV/Vis spectrometer against the blank value (0 nmol phosphate). The mean of the absorptions of the duplicate determination was obtained. The values of the standard were plotted against the amounts of contained phosphate in a calibration line. This served to quantify the amount of phosphate and lipid, respectively, contained in the samples. The P/L ratio was obtained by dividing the peptide concentration by the lipid concentration.

### Example 5: Evaluation of the fusion kinetics

The fusion kinetics were obtained in per cent [%] as a function of time [min] using Microsoft Excel. The values of the kinetics of the empty liposomes (without peptide) were subtracted from these values. The fusion values were corrected by the value of the decrease in the fluorescence of NBD by addition under the influence of the detergent Triton X-100 (2.8 %). In the evaluation of the fusion kinetics, the value thereof was read off after one hour (extent of fusion). To determine the initial rate of fusion, the kinetics in the first 10 minutes were fitted using a polynomial and the first derivation was obtained (Origin 7.0 software, OriginLabs). The value at t = 1 min was read off and used for the comparison of the peptides (unit: %/min).

### Example 6: Measurement or liposome fusion with histidine peptides

For measurement of the fusogenicity of histidine peptides at various pH values, these were first integrated into lipid films as standard. Rehydration was carried out using buffers in the pH range of from 4 to 7.5. In this, 20 mM Tris-HCl was replaced by 20 mM citric acid/sodium citrate at pH 4 to 5. Between pH 5.5 and 7.5, 20 mM Tris-HCl was replaced by 20 mM Tris-maleate-NaOH. The concentrations of NaCl (150 mM), EDTA (0.1 mM) and DTT (5 mM) remained unchanged.

### Example 7: Transport of liposomes containing a peptide and a fat-soluble therapeutically active substance into a cell

Liposomes are prepared as described in Example 3 or 4. To the liposomes 0.8 mol% (based on the sum of the substance amounts of the lipids employed) marker lipids L-α-phosphatidylethanolamine-N-(lissamine rhodamine B sulfonyl) (Rh-PE) und 0,8 mol% (based on the sum of the substance amounts of the lipids employed) L-α-phosphatidylethanolamine-N-(4-nitrobenzo-2-oxa-1,3-diazole) (NBD-PE) are admixed. The mixtures are dried on the vessel wall as a film, and liposomes are prepared from this by the methods in Example 3 or 4.

The liposomes are added to the culture medium of mammalian cells and the cells are incubated in this at 37 °C. Liposomes, which have not fused with the cells during the incubation period are removed by washing the cells several times. The fusion, which has taken place is detected by recording a fluorescence spectrum. The fusion, which has taken place manifests itself by the decrease in the fluorescence resonance energy transfer between NBD-PE and Rh-PE, by an increase in the fluorescence at 530 nm.

### Example 8: Transport of liposomes containing a peptide and a water-soluble therapeutically active substance into a cell

Liposomes are prepared as described in Example 3 or 4. The model substance carboxyfluorescein (CF) is admixed to these in a self-quenching concentration (100 mM) in resuspending buffer. CF, which has not been incorporated into liposomes, is removed by gel permeation chromatography. The liposomes filled with CF are added to the culture medium of mammalian cells and the cells are incubated at 37 °C. Non-fused liposomes are removed by washing the cells several times. If the CF is released inside the cell after fusion of the liposomes with the cell, it is diluted in the cytosol, which leads to an increase in the fluorescence at 558 nm. This is detected by means of fluorescence microscopy.

### References

1. Miranda, L.P. and Alewood, P.F. (1999): Accelerated chemical synthesis of peptides and small proteins. Proc Natl Acad Sci U S A 96, 1181-6.
2. New, R.R.C.E. (1990): Liposomes - A practical approach. Oxford University Press.
3. Struck, D.K., Hoekstra, D. and Pagano, R.E. (1981): Use of resonance energy transfer to monitor membrane fusion. Biochemistry 20, 4093-9.
4. Langosch, D., Brosig, B. and Pipkorn, R. (2001): Peptide mimics of the vesicular stomatitis virus G-protein transmembrane segment drive membrane fusion in vitro. J Biol Chem 276, 32016-21.
5. Olson, F., Hunt, C.A., Szoka, F.C., Vail, W.J. and Papahadjopoulos, D. (1979): Preparation of liposomes of defined size distribution by extrusion through polycarbonate membranes. Biochim Biophys Acta 557, 9-23.
6. Hamilton, R.L., Jr., Goerke, J., Guo, L.S., Williams, M.C. and Havel, R.J. (1980): Unilamellar liposomes made with the French pressure cell: a simple preparative and semiquantitative technique. J Lipid Res 21, 981-92.
7. Kirby, C.J. and Gregoriadis, G. (1984): Preparation of liposomes containing factor VIII for oral treatment of haemophilia. J Microencapsul 1, 33-45.
8. Torchilin, V.P. and Weissig, V. (2003): Liposomes. Oxford University Press 2.
9. Batzri, S. and Korn, E.D. (1973): Single bilayer liposomes prepared without sonication. Biochim Biophys Acta 298, 1015-9.
10. Szoka, F., Jr. and Papahadjopoulos, D. (1978): Procedure for preparation of liposomes with large internal aqueous space and high capture by reverse-phase evaporation. Proc Natl Acad Sci U S A 75, 4194-8.
11. Schurtenberger, P., Mazer, N., Waldvogel, S. and Kanzig, W. (1984): Preparation of monodisperse vesicles with variable size by dilution of mixed micellar solutions of bile salt and phosphatidylcholine. Biochim Biophys Acta 775, 111-4.
12. Brunner, J., Skrabal, P. and Hauser, H. (1976): Single bilayer vesicles prepared without sonication. Physico-chemical properties. Biochim Biophys Acta 455, 322-31.
13. Holloway, P.W. (1973): A simple procedure for removal of Triton X-100 from protein samples. Anal Biochem 53, 304-8.

## Claims

1. Peptide having an amino acid sequence of the formula I:
(A)ₓ(B)_{y}(C)_{z}(B)_{y}(A)ₓ
wherein
A is an essentially hydrophilic amino acid, preferably glycine (G), lysine (K) or arginine (R), particularly preferred histidine (H);
X is 0 or 1;
B is histidine (H);
Y is an integer from 1 to 6; or
Y is an integer from 0 to 5; when A is histidine (H) and X is the factor 1;
C is a essentially hydrophobic amino acid, preferably selected from the group consisting of valine (V), leucine (L), Isoleucine (I), proline (P), glycine (G), cysteine (C), tryptophane (W), alanine (A), phenylalanine (F) and methionine (M); and
Z is an integer from 7 to 30.

2. Peptide according to claim 1, wherein the amino acid sequence comprises from at least 9 to 38 consecutive amino acids comprising at least 1 histidine residue at the N-terminus as well as at the C-terminus and from at least 6 to 30 essentially hydrophobic amino acids between said histidine residue.

3. Peptide according to claim 2, wherein said peptide comprises 3 histidine residues at the N-terminus as well as at the C-terminus.

4. Peptide according to claim 2 or 3, wherein said peptide comprises 17 essentially hydrophobic amino acids.

5. Peptide according to any of the preceding claims, wherein the essentially hydrophobic amino acids are selected from the group consisting of valine (V), leucine (L), Isoleucine (I), proline (P), glycine (G), cysteine (C), tryptophane (W), alanine (A), phenylalanine (F) and methionine (M).

6. Peptide according to any of the preceding claims, wherein said peptide comprises the amino acid sequence shown in one of the figures 1 to 30.

7. Peptide according to any of the preceding claims, wherein said peptide is incorporated into a spherical lipid bilayer.

8. Peptide according to claim 7, wherein the spherical lipid bilayer is a liposome.

9. Peptide according to claim 7 or 8, wherein the liposome is selected from the group consisting of phosphateidylserine (PS), phosphateidylethanolamine (PE), palmityloleyl-phosphateidylcholine (POPC), dioleyl-phosphateidylethanolamine (DOPE), dioleyl-phosphateidylserine (DOPS), stearyl-docosahexanyl-PE, stearyl-docosahexanyl-PS, lipofectine, lipofectamine, lipofectamine 2000 and colipids, such as POPE.

10. Complex comprising at least one peptide according to any of claims 1 to 9, at least one therapeutically active substance and a spherical lipid bilayer.

11. Complex according to claim 10, wherein the spherical lipid bilayer is a liposome.

12. Method for transfecting a cell comprising the following steps:
a. mixing a peptide of any of claims 1 to 9 with a therapeutically active substance and a spherical lipid bilayer to form a complex of claim 10 or 11,
b. contacting a cell with the complex from step a.

13. Use of a peptide of any of claims 1 to 9 or a complex of claim 10 or 11 for effecting and/or enhancing of cell transfection efficiency.

14. Use of a peptide of any of claims 1 to 9 or a complex of claim 10 or 11 for stimulating and/or effecting a pH-switchable membrane fusion between the membrane of a spherical lipid bilayer and the membrane of a cell.

15. Kit comprising at least one peptide of any of claims 1 to 9, at least one therapeutically active substance and a spherical lipid bilayer.

16. Kit according to claim 15, wherein the spherical lipid bilayer is a liposome.
